(19) Europäisches Patentamt
European Patent Office
Office européen des brevets

(11) **EP 4 580 800 B1**

(12) **EUROPEAN PATENT SPECIFICATION**

(45) Date of publication and mention
of the grant of the patent:
**01.04.2026 Bulletin 2026/14**

(21) Application number: **23764262.4**

(22) Date of filing: **28.08.2023**

(51) International Patent Classification (IPC):
**B01J 8/18** (2006.01)    **C07C 45/60** (2006.01)
**C07C 47/19** (2006.01)

(52) Cooperative Patent Classification (CPC):
(C-Sets available)
**C07C 45/60**; Y02E 50/10                (Cont.)

(86) International application number:
**PCT/EP2023/073516**

(87) International publication number:
**WO 2024/046969 (07.03.2024 Gazette 2024/10)**

(54) **THERMOLYTIC FRAGMENTATION OF SUGARS**

THERMOLYTISCHE FRAGMENTIERUNG VON ZUCKERN

FRAGMENTATION THERMOLYTIQUE DE SUCRES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **29.08.2022 EP 22192644**

(43) Date of publication of application:
**09.07.2025 Bulletin 2025/28**

(73) Proprietor: **Topsoe A/S
2800 Kgs. Lyngby (DK)**

(72) Inventors:
• **LARSEN, Morten Boberg
2765 Smørum (DK)**
• **THORHAUGE, Max
2730 Herlev (DK)**
• **OSMUNDSEN, Christian Mårup
2820 Gentofte (DK)**
• **BEIER, Matthias Josef
3460 Birkerød (DK)**

(74) Representative: **Topsoe A/S
Haldor Topsøes Allé 1
2800 Kgs. Lyngby (DK)**

(56) References cited:
**WO-A1-02/40436        WO-A1-2017/216311
WO-A1-2021/032590**

• **OSMUNDSEN: "Catalytic Conversion of
Carbohydrates", DEPARTMENT OF PHYSICS
TECHNICAL UNIVERSITY OF DENMARK, 1
January 2013 (2013-01-01), XP055238243,
Retrieved from the Internet <URL:http://orbit.dtu.
dk/files/54426932/Thesis final.pdf> [retrieved on
20151223]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 45/60, C07C 47/19**

## Description

## Field of the invention

The present invention relates to a method for thermolytic fragmentation of a sugar into C1-C3 oxygenates. The present invention also relates to a system for thermolytic fragmentation of a sugar into C1-C3 oxygenates. The method is suitable for industrial scale production.

## Background

Biomass is of particular interest as a raw material due to its potential for supplementing and possibly replacing petroleum as a feedstock for the preparation of commercial chemicals. In recent years, various technologies for exploiting biomass have been investigated.

Carbohydrates represent a large fraction of biomass, and various strategies for their efficient use as a feedstock for the preparation of commercial chemicals are being established. These strategies include various fermentation-based processes, pyrolysis, and different processes employing catalysis such as hydrogenolysis, conversion via retro-aldol chemistry, and various acid catalysed dehydration processes.

Examples of chemicals produced from biomass include: small alcohols such as ethanol, propanol, and butanol, monomers such as ethylene glycol, ethylene, propylene, butadiene, isoprene, furandicarboxylic acid, succinic acid, lactic acid and lactide, acrylic acid, epichlorohydrin, and vinyl glycolic acid. Also included are diols, such as 1,2-propanediol, 1,2-butanediol, 2,3-butanediol, 1,2-pentanediol, 1,2-hexanediol, and sorbitol, glycerol, glycolaldehyde, pyruvaldehyde, and levulinic acid. Other products such as substitute natural gas, synthesis gas, and biofuels are also included.

Within the field of pyrolysis, efforts have been focused on using feedstocks based on solid biomass and other lignocellulosic materials for producing the above chemicals.

Some efforts have been made to use sugars as feedstock for producing food browning materials, which comprise a large amount of glycolaldehyde (also termed hydroxyacetaldehyde) as the key browning agent.

Fluidised bed reactors comprising heat carrying particles are used for processing a variety of feedstocks. They can be operated in a number of different fluidisation regimens. The preferred regime is selected depending on the feedstock in question and the desired chemistry to be obtained, which gives rise to a large number of different reactor configurations for fluidised bed reactors.

For conversion of biomass into bio-oil by pyrolysis, several reactor configurations have been investigated, such as dense phase (i.e. bubbling fluidised bed) and dilute phase (i.e. riser) reactors, as well as radically different reactor types, such as ablative pyrolysis reactors.

Examples of prior art disclosing the use of fluidised bed reactors include US 5,397,582 (Underwood), US 7,094,932 (Majerski), WO 2014/131764, WO 2012/115754, US 5,302,280 (Lomas), and WO 2017/216311 (Larsen et al.). WO2021/032590A1 discloses a process for the thermolytic fragmentation of a sugar into a composition of C1-C3 oxygenates. This document also discloses that the cooled heat carrying particles (by contact with feedstock solution) leave the fragmentation zone with 350-550 °C.

There remains a need for improved methods and systems for thermolytic fragmentation of a sugar into C1-C3 oxygenates, in particular which are suitable for industrial scale production.

## Summary of the invention

According to an aspect of the present invention, there is provided a process for thermolytic fragmentation of a sugar into C1-C3 oxygenates, the process comprising the steps of:

　　a) providing an aqueous feedstock solution comprising the sugar;
　　b) providing a fluidised bed fragmentation reactor for thermolytically fragmenting the sugar and comprising fluidisable heat carrying particles;
　　c) introducing the feedstock solution into the reactor to thermolytically fragment the sugar to provide a fragmentation product comprising the C1-C3 oxygenates, wherein the temperature of the fragmentation product at an outlet of the reactor is at least 400°C;
　　d) cooling the fragmentation product downstream of the reactor to a cooling temperature of from 230°C to 390°C; and
　　e) separating solids from the fragmentation product cooled to the cooling temperature, wherein the solids comprise solids selected from fluidisable heat carrying particles, fragments of the fluidisable heat carrying particles, by-products from the thermolytic fragmentation of the feedstock solution, and mixtures thereof.

The present inventors have surprisingly discovered that by cooling the fragmentation product downstream of the reactor to the cooling temperature, the process provides for improvements in efficiency and design, whilst achieving a good product yield of C1-C3 oxygenates. In particular, by virtue of the downstream cooling step, e.g. rather than including a cooling step within the reactor, the physical unit operation of the cooling step and the reactor can be arranged in different locations from each other. In this way, the reactor can be designed without the need to accommodate the physical unit operation of the cooling step, which reduces design complexity and installation complexity, and improves space-efficiency and ease of handling. With regard to reducing installation complexity, the physical unit operation of the cooling step can be located closer to ground level than the reactor and installed independently of the reactor. With regard to space-efficiency and ease of handling, as the reactor can be designed without the need to accommodate the physical unit operation of the cooling step, the reactor can be more compact and of a lower mass. Moreover, for example, the physical unit operation of the cooling step can be connected to the reactor (e.g. in a modular fashion) once the reactor has been installed. The cooling to the cooling temperature downstream of the reactor unexpectedly led to these advantages as well as a good product yield of C1-C3 oxygenates.

The afore-mentioned advantages are particularly beneficial in the context of industrial production plants, where significant capital expenditure (CAPEX) and operational expenditure (OPEX) can be used for building and maintaining process equipment and in which product yield is a primary consideration.

In the present context, the terms "downstream" and "upstream" are with respect to the direction in which the components flow. For example, the outlet of the reactor is downstream of the point at which the feedstock solution is introduced into the reactor, given that components flow from the point at which the feedstock solution is introduced to the outlet of the reactor.

### a) the feedstock solution

The process comprises providing an aqueous feedstock solution comprising the sugar.

In one aspect, the total sugar content in the feedstock solution is at least 30 wt.%, such as at least 35 wt.%, at least 40 wt.%, at least 45 wt.%, at least 50 wt.%, at least 55 wt.%, at least 60 wt.%, at least 65 wt.%, at least 70 wt.%, at least 75 wt.%, at least 80 wt.%, at least 85 wt.%, at least 90 wt.%, or at least 95 wt.%, based on the total weight of the feedstock solution.

In one aspect, the total sugar content in the feedstock solution is no greater than 99.9 wt.%, such as no greater than 99.5 wt.%, no greater than 99 wt.%, no greater than 95 wt.%, no greater than 85 wt.%, or no greater than 80 wt.%, based on the total weight of the feedstock solution.

In one aspect, the total sugar content in the feedstock solution is from 30 to 99 wt.%, such as from 40 to 90 wt.%, or from 50 to 80 wt.%, based on the total weight of the feedstock solution.

In one aspect, the feedstock solution is a liquid.

In one aspect, the water content in the feedstock solution is at least 20 wt.%, such as at least 25 wt.%, at least 30 wt.%, at least 35 wt.%, at least 40 wt.%, at least 45 wt.%, at least 50 wt.%, based on the total weight of the feedstock solution.

In one aspect, the water content in the feedstock solution is no greater than 70 wt.%, such as no greater than 65 wt.%, no greater than 60 wt.%, no greater than 55 wt.%, no greater than 50 wt.%, or no greater than 45 wt.%, based on the total weight of the feedstock solution.

In one aspect, the total sugar content in the aqueous feedstock solution is from 20 to 70 wt.%, such as from 25 to 60 wt.%, or from 30 to 50 wt.%, based on the total weight of the feedstock solution.

In one aspect, the sugar is a carbohydrate comprising one or more C6 saccharide units and/or C5 saccharide units.

In one aspect, the sugar is a monosaccharide or a disaccharide.

In one aspect, the sugar is selected from sucrose, lactose, xylose, arabinose, ribose, mannose, tagatose, galactose, glucose and fructose.

In one aspect, the feedstock solution may comprise a sugar syrup.

In one aspect, the feedstock solution comprises more than one sugar.

Each sugar may be independently as described herein.

### b) providing a fluidised bed fragmentation reactor for thermolytically fragmenting the sugar and comprising fluidisable heat carrying particles

The process comprises providing a fluidised bed fragmentation reactor for thermolytically fragmenting the sugar and comprising fluidisable heat carrying particles.

As will be understood by those skilled in the art, a fluidised bed reactor is a reactor which accommodates a bed of particles which may be fluidised, e.g. by a fluidisation gas which may be introduced at the base of the reactor. The velocity and physical properties of the fluidisation gas combined with the physical properties of the fluidisable particles may regulate the fluidisation state of the fluidisable particles within the bed. A dense bed/turbulent bed/bubbling bed has a superficial velocity of the fluidisation gas within the reactor of from 0.01 to 2 m/s. A fast bed (also termed a riser/transport reactor) may have a superficial velocity of fluidisation gas within the reactor of from 3 to 22 m/s. The exact velocity range is, however, dependent on the physical properties of the fluidisable particles and the fluidisation gas, and can be determined experimentally or calculated by those skilled in the art.

In one aspect, the fluidised bed fragmentation reactor is operative in the bubbling fluidisation regime, the slugging fluidisation regime, the turbulent fluidisation regime, the fast fluidisation regime, and/or the pneumatic conveying regime.

In one aspect, the fluidised bed fragmentation reactor is operative in the fast fluidisation regime. Such a reactor is also termed a "riser type reactor". Thus, in one aspect, the reactor is a riser type reactor.

In one aspect, the reactor comprises a riser.

In one aspect, the riser extends vertically.

In one aspect, the riser (e.g. a lower part of the riser) comprises a fluidisation gas inlet.

In one aspect, the riser (e.g. a lower part of the riser) comprises a fluidisable particle inlet.

In one aspect, the riser (e.g. a lower part of the riser) comprises a feedstock inlet.

In one aspect, the fluidisable particle inlet is provided downstream of the fluidisation gas inlet.

In one aspect, the feedstock solution inlet is provided downstream of the fluidisable particle inlet.

In one aspect, the fluidisable heat carrying particles form a dense phase fluidised bed in a zone between the fluidisable particle inlet and the feedstock solution inlet.

In one aspect, the riser (e.g. a lower part of the riser) comprises a fluidisable particle and fluidisation gas inlet. In this way, the fluidisable heat carrying particles and the fluidisation gas can be introduced into the reactor via a common inlet, e.g. sequentially or at the same time.

In one aspect, the feedstock solution inlet is provided downstream of the fluidisable particle and fluidisation gas inlet.

In one aspect, the method comprises introducing fluidisable heat carrying particles into the reactor.

In one aspect, the method comprises introducing fluidisable heat carrying particles into the reactor via the fluidisable particle inlet.

In one aspect, the method comprises introducing fluidisable heat carrying particles into the reactor via the fluidisable particle and fluidisation gas inlet.

In one aspect, the temperature of the fluidisable heat carrying particles within the reactor is at least 400°C, such as at least 450°C, at least 500°C, at least 550°C, at least 600°C, or at least 650°C.

In one aspect, the temperature of the fluidisable heat carrying particles within the reactor is no greater than 800°C, such as no greater than 700°C, or no greater than 650°C.

In one aspect, the temperature of the fluidisable heat carrying particles within the reactor is from 400 to 800°C, such as from 500 to 800°C, from 450 to 650°C, or from 550 to 650°C.

In one aspect, the temperature of the fluidisable heat carrying particles at the fluidisable particle inlet (or the fluidisable particle and fluidisation gas inlet) is at least 400°C, such as at least 450°C, at least 500°C, at least 550°C, at least 600°C, or at least 650°C.

In one aspect, the temperature of the fluidisable heat carrying particles at the fluidisable particle inlet (or the fluidisable particle and fluidisation gas inlet) is no greater than 800°C, such as no greater than 700°C, or no greater than 650°C.

In one aspect, the temperature of the fluidisable heat carrying particles at the fluidisable particle inlet (or the fluidisable particle and fluidisation gas inlet) is from 400 to 800°C, such as from 500 to 800°C, from 450 to 650°C, or from 550 to 650°C.

In one aspect, the pressure at the outlet of the reactor is at least 0.9 bara, such as at least 1 bara, at least 1.2 bara, at least 1.4 bara, at least 1.6 bara, at least 1.7 bara, at least 1.8 bara, at least 2 bara, or at least 2.2 bara.

In one aspect, the pressure at the outlet of the reactor is no greater than 8 bara, such as no greater than 6 bara, no greater than 5 bara, no greater than 4 bara, no greater than 3.5 bara, no greater than 3 bara, no greater than 2.9 bara, or no greater than 2.8 bara.

In one aspect, the pressure at the outlet of the reactor is from 0.9 to 8 bara, such as from 1 to 6 bara, from 1.1 to 5 bara, from 1.2 to 4 bara, from 1.5 to 3 bara, from 1.6 to 2.9 bara, from 1.7 to 2.8 bara, from 1.8 to 2.8 bara, from 2 to 2.8 bara, or from 2.2 to 2.8 bara.

In one aspect, the fluidisable heat carrying particles are selected from sand, mullite, silica, glass, alumina, silica-alumina, steel, and silicon carbide.

In one aspect, the mean particle size of the fluidisable heat carrying particles is from 20 to 400 $\mu$m, such as from 20 to 300 $\mu$m, or from 20 to 200 $\mu$m. The particle size distribution can be measured by laser diffraction spectroscopy, microscopy, electrical zone sensing. The mean particle size can be subsequently calculated from the particle size distribution. The mean particle size may be the Sauter mean particle size. Those skilled in the art will appreciate that other mean particles sizes are envisaged.

As will be appreciated by those skilled in the art, the flow of the fluidisable heat carrying particles within the reactor may be adjusted relative to the flow of the feedstock solution so as to provide the desired amount of heat to the feedstock solution. Fluidisable particles with a high heat capacity may require a lower mass flow rate than fluidisable particles with a lower heat capacity.

**c) introducing the feedstock solution into the reactor to thermolytically fragment the sugar to provide a fragmentation product comprising the C1-C3 oxygenates, wherein the temperature of the fragmentation product at an outlet of the reactor is at least 400°C**

The method comprises introducing the feedstock solution into the reactor to thermolytically fragment the sugar to provide a fragmentation product comprising the C1-C3 oxygenates, wherein the temperature of the fragmentation product at an outlet of the reactor is at least 400°C.

The thermolytic fragmentation of sugar is an endothermic reaction, mainly due to evaporation of any liquid in the feedstock solution and the reaction enthalpy of thermolytic fragmentation of the sugar to the oxygenates.

In one aspect, the temperature of the fragmentation product at an outlet of the reactor is at least 420°C, such as at least 430°C, at least 440°C, at least 450°C, at least 460°C, or at least 470°C.

In one aspect, the temperature of the fragmentation product at an outlet of the reactor is no greater than 550°C, such as no greater than 530°C, no greater than 520°C, no greater than 510°C, no greater than 500°C, or no greater than 490°C.

In one aspect, the temperature of the fragmentation product at an outlet of the reactor is from 400 to 600°C, such as from 420 to 550°C, from 450 to 510°C, from 460 to 500°C, or from 470 to 490°C.

In one aspect, in step c) the feedstock solution is introduced into the reactor at a rate of at least 1000 kg/h (kilogrammes per hour). In one aspect, in step c) the feedstock solution is introduced into the reactor at a rate of at least 2000 kg/h. In one aspect, in step c) the feedstock solution is introduced into the reactor at a rate of at least 5000 kg/h. In one aspect, in step c) the feedstock solution is introduced into the reactor at a rate of at least 10000 kg/h. In one aspect, in step c) the feedstock solution is introduced into the reactor at a rate of at least 15000 kg/h. In one aspect, in step c) the feedstock solution is introduced into the reactor at a rate of at least 20000 kg/h.

There may be no upper limit as to the rate at which feedstock solution is introduced into the reactor in step c). In one aspect, in step c) the feedstock solution is introduced into the reactor at a rate of no greater than 1000000 kg/h. In one aspect, in step c) the feedstock solution is introduced into the reactor at a rate of no greater than 100000 kg/h.

**Fluidisation gas**

In one aspect, the process comprises introducing a fluidisation gas into the reactor.

As will be understood by those skilled in the art, the fluidisation gas fluidises the fluidisable heat carrying particles in the reactor.

In one aspect, the process comprises introducing a fluidisation gas into the reactor via the fluidisation gas inlet.

In one aspect, the process comprises introducing a fluidisation gas into the reactor via the fluidisable particle and fluidisation gas inlet.

When the feedstock solution encounters the fluidisable heat carrying particles in the reactor, a vaporisation zone is formed in which the liquid evaporates and gaseous products are generated via sugar fragmentation. This results in an increase in the superficial velocity of the gas products thereby entraining the fluidisable heat carrying particles. Accordingly, downstream of the feedstock solution inlet, the fluidisable heat carrying particles and the feedstock solution form a fast bed above the vaporisation zone.

In one aspect, step c) comprises introducing the feedstock solution and the fluidisation gas into the reactor to thermolytically fragment the sugar to provide the fragmentation product.

In one aspect, in step c) the feedstock solution is entrained in the fluidisation gas.

In one aspect, the fluidisation gas comprises water (e.g. steam).

In one aspect, the fluidisation gas consists essentially of water (e.g. steam).

In one aspect, the fluidisation gas consists of water (e.g. steam).

Herein, "water" and "steam" may be used interchangeably.

In one aspect, in step c) the fluidisation gas is introduced into the reactor at a rate of at least 100 kg/h. In one aspect, in step c) the fluidisation gas is introduced into the reactor at a rate of at least 500 kg/h. In one aspect, in step c) the fluidisation gas is introduced into the reactor at a rate of at least 1000 kg/h. In one aspect, in step c) the fluidisation gas is introduced into the reactor at a rate of at least 2000 kg/h. In one aspect, in step c) the fluidisation gas is introduced into the reactor at a rate of at least 4000 kg/h. In one aspect, in step c) the fluidisation gas is introduced into the reactor at a rate of at least 5000 kg/h.

There may be no upper limit as to the rate at which the fluidisation gas is introduced into the reactor in step c). In one aspect, in step c) the fluidisation gas is introduced into the reactor at a rate of no greater than 50000 kg/h. In one aspect, in step c) the fluidisation gas is introduced into the reactor at a rate of no greater than 20000 kg/h.

**Atomisation gas**

In one aspect, the process comprises introducing an atomisation gas into the reactor.

As will be understood by those skilled in the art, the atomisation gas is used to atomise the feedstock solution introduced into the reactor. The term atomising is meant to refer to turning a liquid into small droplets.

In one aspect, the atomisation gas and the feedstock solution are introduced into the reactor such that the feedstock solution is atomised by the atomisation gas.

In one aspect, the atomisation gas comprises water (e.g. steam).

In one aspect, the atomisation gas consists essentially of water (e.g. steam).

In one aspect, the atomisation gas consists of water (e.g. steam).

In one aspect, in step c) the atomisation gas is introduced into the reactor at a rate of at least 50 kg/h. In one aspect, in step c) the atomisation gas is introduced into the reactor at a rate of at least 100 kg/h. In one aspect, in step c) the atomisation gas is introduced into the reactor at a rate of at least 200 kg/h. In one aspect, in step c) the atomisation gas is introduced into the reactor at a rate of at least 500 kg/h. In one aspect, in step c) the atomisation gas is introduced into the reactor at a rate of at least 800 kg/h. In one aspect, in step c) the atomisation gas is introduced into the reactor at a rate of at least 1000 kg/h. In one aspect, in step c) the atomisation gas is introduced into the reactor at a rate of at least 1200 kg/h.

There may be no upper limit as to the rate at which the atomisation gas is introduced into the reactor in step c). In one aspect, in step c) the atomisation gas is introduced into the reactor at a rate of no greater than 20000 kg/h. In one aspect, in step c) the atomisation gas is introduced into the reactor at a rate of no greater than 10000 kg/h.

**Water introduced**

The process comprises introducing water into the reactor.

The water may be introduced in the form of one or both of gas (steam) and liquid. The water may be introduced in various different ways, and by one or more different means, including, for example, via the feedstock solution and/or via the fluidisation gas and/or via the atomisation gas).

In one aspect, water is introduced into the reactor via the atomisation gas.

In one aspect, water is introduced into the reactor via the feedstock solution.

**The fragmentation product**

The fragmentation product comprises C1-C3 oxygenates resulting from the thermolytic fragmentation of the feedstock solution. The fragmentation product provided in step (c) is a crude product. The fragmentation product provided in step (c) comprises the solids.

In one aspect, in step c) the fragmentation product is a solids-dense fragmentation product.

In one aspect, the solids-dense fragmentation product comprises the C1-C3 oxygenates and the solids.

In one aspect, step e) comprises separating solids from the solids-dense fragmentation product cooled to the cooling temperature to provide a solids-lean fragmentation product, wherein the solids comprise solids selected from the fluidisable heat carrying particles, fragments of the fluidisable heat carrying particles, by-products from the thermolytic fragmentation of the feedstock solution, and mixtures thereof.

In one aspect, the solids-lean fragmentation product comprises the C1-C3 oxygenates.

In one aspect, the solids-lean fragmentation product comprises a lower amount of the solids than the solids-dense fragmentation product.

The terms "lean" in "solids-lean" and "dense" in "solids-dense" are relative to each other.

In one aspect, the C1-C3 oxygenates comprise one, more, or each of formaldehyde (C1), glycolaldehyde (C2), glyoxal (C2), pyruvaldehyde (C3), and acetol (C3). For most uses, the C2 and the C3 oxygenates are most valuable.

In one aspect, the fragmentation product comprises a mixture of two or more of C1-C3 oxygenates. The mixture may be interchangeably referred to as a C1-C3 oxygenate mixture, a C1-C3 oxygenate product, or C1-C3 oxygenates.

In one aspect, the fragmentation product comprises glycolaldehyde.

In one aspect, the fragmentation product includes a major portion of glycolaldehyde.

In one aspect, the fragmentation product comprises glycolaldehyde in an amount of at least 10 wt.%, such as at least 20 wt.%, at least 30 wt.%, at least 40 wt.%, at least 50 wt.%, at least 60 wt.%, or at least 70 wt.%, based on the total weight of the fragmentation product.

In one aspect, the fragmentation product comprises one or more of glycolaldehyde or glyoxal in an amount of at least 10 wt.%, such as at least 20 wt.%, at least 30 wt.%, at least 40 wt.%, at least 50 wt.%, at least 60 wt. %, at least 70 wt. %, or at least 80 wt. %, based on the total weight of the fragmentation product.

In one aspect, the fragmentation product comprises one or more of pyruvaldehyde or acetol in an amount of at least 3 wt. %, such as at least 5 wt. %, or at least 7 wt. %, based on the total weight of the fragmentation product.

In one aspect, the total amount of C1-C3 oxygenates in the fragmentation product is no greater than 99 wt.%, such as no greater than 95 wt.%, or no greater than 90 wt.%, based on the total weight of the fragmentation product.

In one aspect, "based on the total weight of the fragmentation product" refers to the fragmentation product provided in step (c) (e.g. the solids-dense fragmentation product).

In one aspect, "based on the total weight of the fragmentation product" refers to the fragmentation product following step (e) (e.g. the solids-lean fragmentation product).

In one aspect, "based on the total weight of the fragmentation product" refers to the fragmentation product not including the solids.

**d) cooling the fragmentation product downstream of the reactor to the cooling temperature**

The process comprises cooling the fragmentation product downstream of the reactor to a cooling temperature of from 230°C to 390°C.

The "cooling temperature" corresponds to an average temperature of the fragmentation product after step d) of cooling.

In one aspect, the cooling temperature is from 250 to 390°C.

In one aspect, the cooling temperature is from 260 to 385°C.

In one aspect, the cooling temperature is from 270 to 380°C.

In one aspect, the cooling temperature is from 300 to 375°C.

In one aspect, the cooling temperature is from 330 to 370°C.

In one aspect, step d) comprises indirectly cooling the fragmentation product to the cooling temperature.

In one aspect, step d) comprises cooling the fragmentation product to the cooling temperature using a cooling device.

In one aspect, the cooling device is a heat exchanger.

In one aspect, the cooling device is an indirect heat exchanger.

In one aspect, step d) is the earliest step of cooling the fragmentation product.

In one aspect, no step of cooling the fragmentation product is performed within the reactor.

In one aspect, no step of cooling the fragmentation product is performed prior to step d).

In one aspect, step d) is performed at a location below the location at which the reactor is provided (with respect to sea level).

In one aspect, the cooling device (e.g. the heat exchanger) is provided at a location below the location at which the reactor is provided (with respect to sea level).

In one aspect, a lowermost surface of the cooling device (e.g. the heat exchanger) is provided at a location below the location at which a lowermost surface of the reactor is provided (with respect to sea level).

In one aspect, no cooling step is conducted within the reactor.

**e) separating solids from the fragmentation product cooled to the cooling temperature, wherein the solids comprise solids selected from the fluidisable heat carrying particles, fragments of the fluidisable heat carrying particles, by-products from the thermolytic fragmentation of the feedstock solution, and mixtures thereof**

The process comprises separating solids from the fragmentation product cooled to the cooling temperature, wherein the solids comprise solids selected from the fluidisable heat carrying particles, fragments of the fluidisable heat carrying particles, by-products from the thermolytic fragmentation of the feedstock solution, and mixtures thereof.

In one aspect, step e) comprises separating at least 50 wt.%, at least 60 wt.%, at least 70 wt.%, at least 80 wt.%, at least 90 wt.%, at least 95 wt.%, or at least 99 wt.% of the solids from the fragmentation product cooled to the cooling temperature, based on the total weight of the solids.

In one aspect, step e) comprises separating no greater than 99.999 wt.%, such as no greater than 99.99 wt.%, no greater than 99.9 wt.%, no greater than 99.5 wt.%, or no greater than 99 wt.% of the solids from the fragmentation product cooled to the cooling temperature, based on the total weight of the solids.

In one aspect, step e) comprises separating substantially all of the solids from the fragmentation product cooled to the cooling temperature.

In one aspect, step e) comprises separating the solids from the fragmentation product cooled to the cooling temperature using a physical separation device.

In one aspect, the physical separation device is a particle separator.

In one aspect, the physical separation device is a low volume separator.

In one aspect, the physical separation device is a change of direction separator.

In one aspect, the physical separation device is a cyclone.

In one aspect, the physical separation device is a filter.

In one aspect, the filter is selected from: a metal filter and a ceramic filter.

In one aspect, the filter comprises a filter element. The filter element can be considered as the material through which the fragmentation product comprising the solids (the filtration medium) is filtered, thereby to separate the solids from the fragmentation product.

In one aspect, the filter element comprises a filtration surface. The filtration surface can also be referred to as a filtration area. The filtration surface may be considered as an outer surface of the filter element. The filtration surface may be considered as the interface through which the fragmentation product comprising the solids enters the filter element in use. The filtration surface may be for separating the solids from the fragmentation product. In this way, the solids may collect on the filtration surface in use.

In one aspect, the filter element comprises a porous matrix.

In one aspect, the porous matrix is selected from; a porous fibrous matrix; a porous mesh matrix; a porous matrix comprising sintered spheres; a porous metal matrix (e.g. a sintered porous metal matrix); a porous ceramic matrix; and combinations thereof.

In one aspect, the porous matrix is a porous fibrous matrix. In one aspect, the porous fibrous matrix is a porous fibrous metal matrix. In one aspect, the porous fibrous matrix is a sintered porous fibrous metal matrix.

In one aspect, the filter comprises a plurality of the filter elements. Each of the filter elements may be independently as described herein.

In one aspect, the filter is a candle filter.

In one aspect, the filter is a bag filter.

In one aspect, the filter has an efficiency of at least 99.9% for particles having a size of at least 50 micrometres, such as at least 30 micrometres, at least 20 micrometres, or at least 10 micrometres.

In one aspect, multiple of the physical separation devices are employed. Each of the physical separation devices may be independently as described herein.

**Residence time**

In one aspect, the mean residence time of the fragmentation product between step d) and step e) is no greater than 50 s, such as no greater than 45 s, no greater than 40 s, no greater than 35 s, no greater than 30 s, no greater than 25 s, or no greater than 20 s.

In one aspect, the mean residence time of the fragmentation product between step d) and step e) is at least 0.1 s, such as at least 1 s, at least 3 s, at least 5 s, at least 8 s, at least 10 s, at least 12 s, or at least 15 s.

In one aspect, the mean residence time of the fragmentation product between step d) and step e) is from 0.1 to 50 s, such as from 1 to 40 s, or from 2 to 20 s.

"Between step d) and step e)" means between the end of step d) and the start of step e). Where a cooling device is used in step d), "between step d) and step e)" means between the exit of the fragmentation product from the cooling device and step e). Where a separation device is used in step e), "between step d) and step e)" means between step d) and entry of the fragmentation product into the separation device. Where a cooling device is used in step d) and a separation device is used in step e), "between step d) and step e)" means between the exit of the fragmentation product from the cooling device and entry of the fragmentation product into the separation device.

In one aspect, the mean residence time of the fragmentation product in step e) is from 5 to 50 s, such as from 8 to 30 s, from 10 to 25 s, from 14 to 22 s, from 15 to 20 s, 16 to 17 s.

"In step e)" means the duration of the separating process in step e). Where a physical separation device is used in step e), "in step e)" means from entry of the fragmentation product into the physical separation device to exit of the fragmentation product from the physical separation device. For example, where a filter is used in step e), "in step e)" means from entry of the fragmentation product into the filter to exit of the fragmentation product from the filter.

## Superficial velocity

In one aspect, where a filter is used in step e), the superficial velocity of the fragmentation product at the filtration surface of the filter is from 0.1 to 3 cm/s, such as from 0.15 to 2.5 cm/s, from 0.2 to 2 cm/s, 0.25 to 1.5 cm/s, 0.3 to 1 cm/s, 0.3 to 0.75 cm/s, from 0.35 to 0.6 cm/s, or from 0.4 to 0.5 cm/s.

Those skilled in the art would know how to calculate the surface filtration velocity.

## The solids

The solids comprise solids selected from the fluidisable heat carrying particles, fragments of the fluidisable heat carrying particles, by-products from the thermolytic fragmentation of the feedstock solution, and mixtures thereof.

As the fluidisable heat carrying particles are fluidised within the reactor, an amount of the particles can be transported out of the reactor, e.g. via an outlet of the reactor. The fragments of the particles can be formed by abrasion or breakage of the particles, e.g. by mechanical interaction between the particles themselves and/or between the particles and the reactor.

The by-products can be viscous and/or sticky. Without removal of the by-products, these can adhere to the filter (when present) and/or equipment downstream of the reactor. For example, when a filter is used, the by-products can form an increasingly large, sticky filter cake on the filter over time, such that, eventually, the filter cake needs to be removed.

In one aspect, the solids substantially comprise solids selected from the fluidisable heat carrying particles, fragments of the fluidisable heat carrying particles, by-products from the thermolytic fragmentation of the feedstock solution, and mixtures thereof.

In one aspect, the solids consist essentially of solids selected from the fluidisable heat carrying particles, fragments of the fluidisable heat carrying particles, by-products from the thermolytic fragmentation of the feedstock solution, and mixtures thereof.

In one aspect, the solids consist of solids selected from the fluidisable heat carrying particles, fragments of the fluidisable heat carrying particles, by-products from the thermolytic fragmentation of the feedstock solution, and mixtures thereof.

In one aspect, the process is suitable for processing amounts of sugar of greater than 1,000 tons per year per fragmentation reactor, such as greater than 5000 tons per year per fragmentation reactor, greater than 10000 tons per year per fragmentation reactor, greater than 50000 tons per year per fragmentation reactor, greater than 100000 tons per year per fragmentation reactor, or greater than 1000000 tons per year per fragmentation reactor, based on weight of dry sugar.

In one aspect, the process is suitable for processing amounts of sugar of no greater than 10000000 tons per year per fragmentation reactor, such as no greater than 5000000 tons per year per fragmentation reactor, or no greater than 2000000 tons per year per fragmentation reactor, based on weight of dry sugar.

In one aspect, the thermolytic fragmentation process is operated as a continuous process.

## Further process steps

In one aspect, the process comprises one or more further separation steps between step c) and step d), wherein the or each separation between step c) and step d) comprises separating solids from the fragmentation product, wherein the solids comprise solids selected from fluidisable heat carrying particles, fragments of the fluidisable heat carrying particles, by-products from the thermolytic fragmentation of the feedstock solution, and mixtures thereof. In one aspect, the or each separation between step c) and step d) is performed within the reactor.

In one aspect, the or each separation step between step c) and step d) comprises separating at least 50 wt.%, at least 60 wt.%, at least 70 wt.%, at least 80 wt.%, at least 90 wt.%, at least 95 wt.%, or at least 99 wt.% of the solids from the fragmentation product based on the total weight of the solids.

In one aspect, the or each separation step between step c) and step d) comprises separating no greater than 99.9 wt.%, such as no greater than 99.5 wt.%, no greater than 99 wt.%, no greater than 95 wt.%, no greater than 90 wt.%, or no greater than 80 wt.% of the solids from the fragmentation product based on the total weight of the solids.

In one aspect, the process comprises, between step c) and step d), a primary separation and/or a secondary separation. In one aspect, the primary separation and/or the secondary separation are performed within the reactor.

In one aspect, the primary separation comprises separating solids from the fragmentation product, wherein the solids comprise solids selected from fluidisable heat carrying particles, fragments of the fluidisable heat carrying particles, by-products from the thermolytic fragmentation of the feedstock solution, and mixtures thereof.

In one aspect, the primary separation comprises separating at least 50 wt.%, at least 60 wt.%, at least 70 wt.%, at least 80 wt.%, at least 90 wt.%, at least 95 wt.%, or at least 99 wt.% of the solids from the fragmentation product based on the total weight of the solids.

In one aspect, the primary separation comprises separating no greater than 99.9 wt.%, such as no greater than 99.5 wt.%, no greater than 99 wt.%, no greater than 95 wt.%, no greater than 90 wt.% or no greater than 80 wt.% of the solids from the fragmentation product based on the total weight of the solids.

In one aspect, the secondary separation comprises separating solids from the fragmentation product, wherein the solids comprise solids selected from fluidisable heat carrying particles, fragments of the fluidisable heat carrying particles, by-products from the thermolytic fragmentation of the feedstock solution, and mixtures thereof.

In one aspect, the secondary separation comprises separating at least 50 wt.%, at least 60 wt.%, at least 70 wt.%, at least 80 wt.%, at least 90 wt.%, at least 95 wt.%, or at least 99 wt.% of the solids from the fragmentation product based on the total weight of the solids.

In one aspect, the secondary separation comprises separating no greater than 99.9 wt.%, such as no greater than 99.5 wt.%, no greater than 99 wt.%, no greater than 95 wt.%, no greater than 90 wt.%, or no greater than 80 wt.% of the solids from the fragmentation product based on the total weight of the solids.

The one or more further separation steps between step c) and step d) may help to reduce the amount of the solids in the fragmentation product prior to step d). In this way, less energy is wasted in cooling the heat carrying particles in step d).

The process according to the present invention may provide a fragmentation product which is subjected to one or more further processing steps. For example, subsequent to step d), the fragmentation product may be further processed by one or more of chemical conversion (e.g. into another product, e.g. via a hydrogenation reactor), purification (e.g. to reduce the presence of any impurities), and separation (e.g. to provide specific oxygenates or a specific oxygenate mixture).

According to another aspect of the present disclosure there is provided a system for thermolytic fragmentation of a sugar into C1-C3 oxygenates, the system comprising:

a) an aqueous feedstock solution comprising the sugar;
b) a fluidised bed fragmentation reactor configured to thermolytically fragment the sugar and comprising fluidisable heat carrying particles thereby to provide a fragmentation product comprising the C1-C3 oxygenates, wherein the temperature of the fragmentation product at an outlet of the reactor is at least 400°C;
c) means for cooling the fragmentation product downstream of the reactor to a cooling temperature of from 230°C to 390°C; and
d) a separation device configured to separate solids from the fragmentation product cooled to the cooling temperature, wherein the solids comprise solids selected from fluidisable heat carrying particles, fragments of the fluidisable heat carrying particles, by-products from the thermolytic fragmentation of the feedstock solution, and mixtures thereof.

According to another aspect of the present disclosure there is provided a system for thermolytic fragmentation of a sugar into C1-C3 oxygenates, the system comprising:

a) a fluidised bed fragmentation reactor configured to thermolytically fragment the sugar thereby to provide a fragmentation product comprising the C1-C3 oxygenates; and
b) means for cooling the fragmentation product downstream of the reactor to a cooling temperature,

wherein the means for cooling the fragmentation product is provided at a location below the location at which the reactor is provided (with respect to sea level).

In one aspect, the reactor and the means for cooling are in the form of separate physical structures.

According to another aspect of the present disclosure there is provided a system for thermolytic fragmentation of a sugar into C1-C3 oxygenates, the system comprising:

a) a fluidised bed fragmentation reactor configured to thermolytically fragment the sugar thereby to provide a fragmentation product comprising the C1-C3 oxygenates; and

b) means for cooling the fragmentation product downstream of the reactor to a cooling temperature.

In one aspect, the means for cooling is a cooling device. The cooling device may be as defined herein.

BRIEF DESCRIPTION OF THE DRAWINGS

Embodiments of the present invention are explained by way of examples and with reference to the accompanying drawings. The appended drawings illustrate only examples of embodiments of the present invention, and they are therefore not to be considered limiting of its scope, as the invention may admit to other alternative embodiments.

Fig. 1 shows a cross sectional side view of a fragmentation reactor which forms part of a system according to an embodiment of the present disclosure;

Fig. 2 shows a top view of the fragmentation reactor of Fig. 1;

Fig. 3 shows a cross sectional side view of a reheater which forms part of a system according to an embodiment of the present disclosure;

Fig. 4 shows a cross sectional side view of a system according to an embodiment of the present disclosure (not all components of the system are shown), the system comprising a fragmentation reactor in fluid communication with a reheater;

Fig. 5 shows a block diagram of a system according to an embodiment of the present disclosure (not all components of the system are shown);

Fig. 6 shows the temperature profiles of the fluidisable particle inlet, the outlet of the reactor, and the filter of an example;

Fig. 7 shows the carbon yield in glycolaldehyde carbon recovery, and in C1-C3 oxygenates (in total) carbon recovery, as a function of filtration temperature;

Fig. 8 is a schematic diagram of a candle filter; and

Fig. 9 shows a schematic drawing of a fragmentation reactor which forms part of a system according to an embodiment of the present disclosure.

Position numbers

**[0182]**

1. Fragmentation reactor
2. Fragmentation riser
3. First particle separator
4. Second particle separator
5. Cooling section
6. Primary fluidisation gas inlet
7. Fluidisable particle inlet
8. Feedstock and atomisation gas inlet

9. Product outlet
10. Fluidisable particle outlet
11. Reheater
12. Fuel and combustion air inlet
13. Burner chamber
14. Reheater fluidisable particle inlet
15. Reheater riser
16. Reheater fluidisable particle separator
17. Reheater fluidisable particle outlet
18. Reheater gas outlet
19. Second reheater fluidisable particle separator
20. Stripper
21. Secondary fluidisation gas inlet
22. Secondary reheater fluidisation and stripping gas inlet
O'. Outlet of the reactor
100. Heat exchanger
200. Physical separation device (e.g. filter) for gas-/solid filtration
300. Fragmentation reactor
301. Fluidisation gas
302. Feedstock solution
303. Atomisation gas
304. Feedstock and atomisation gas inlet (feed nozzle)
305. First particle separator
306. Recycled heat carrying particles
307. Approximate boundary separating the dense phase and the lean phase
308. Heating arrangement
309. Cooling step
310. Separation step

DETAILED DESCRIPTION

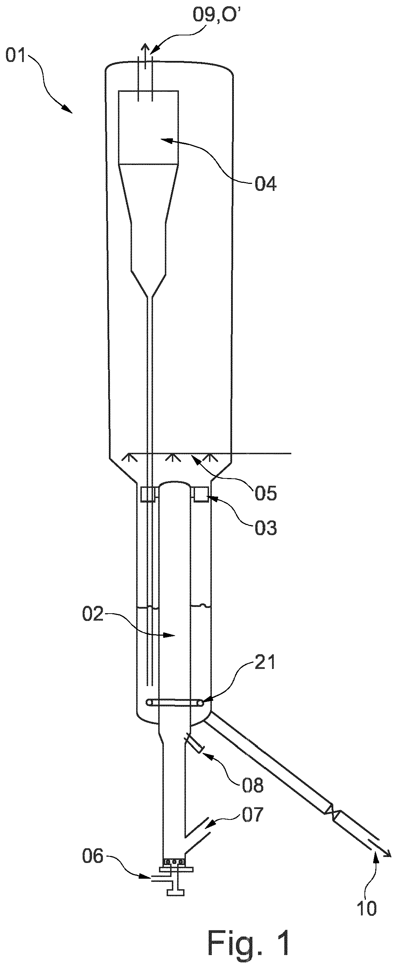

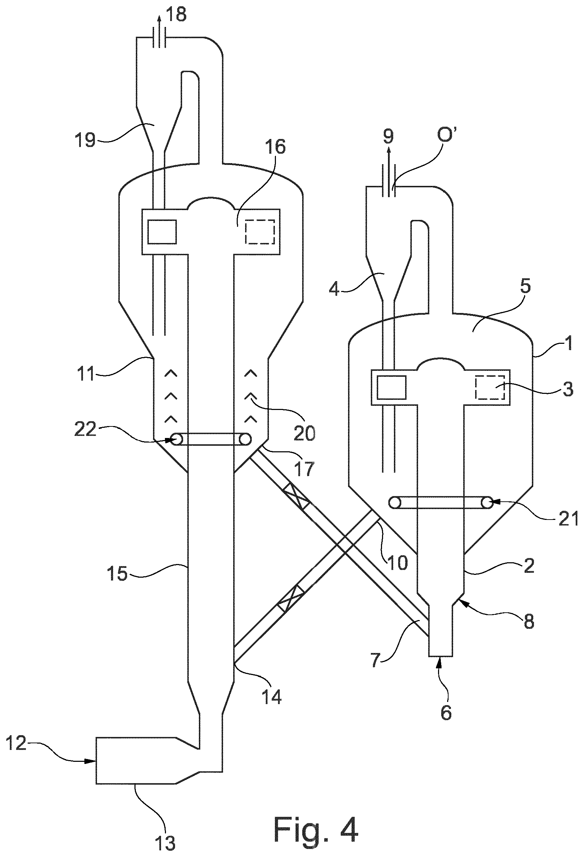

As illustrated in Fig. 1 and Fig 4, the fragmentation reactor 01 is oblong in the vertical direction. Within the fragmentation reactor a riser 02 is provided, which is oblong with a small cross sectional area relative to the height. This facilitates the possibility of a low residence time of the fluidisable heat carrying particles inside the riser 02. In the lower section of the riser 02, a fluidisation gas inlet 06 is provided. The primary fluidisation gas inlet 06 is adapted to provide a fluidisation gas to the riser 02. In the lower section of the riser 02, a fluidisable heat carrying particles inlet 07 is also provided. The reactor 01 further comprises an outlet O'.

The fluidisation gas helps to facilitate the movement of the fluidisable heat carrying particles from the fluidisable heat carrying particle inlet 07 to the feedstock inlet 08 and towards the top of the riser 02. In addition, the fluidisation gas can be used to pre-condition the fluidisable heat carrying particles before the particles are contacted with feedstock solution.

The feedstock and atomisation gas inlet 08 is provided in the riser 02, above the particle and fluidisation gas inlets 06, 07. The feedstock inlet 08 enables the supply of a feedstock solution to the riser 02. As shown in Fig. 1, the feedstock inlet 08 is arranged in the lower section of the riser 02, although its position may vary according to the process demands.

When feedstock solution and fluidisable particles have interacted in the riser 02, they are separated when exiting the riser in a first particle separator 03. In some embodiments, the first particle separator 03 is adapted to provide a fast separation of the fluidisable particles from the fragmentation product (comprising C1-C3 oxygenates) as such a fast separation is highly advantageous to the process. Hence, the particle separator 03 can be of a low residence time type.

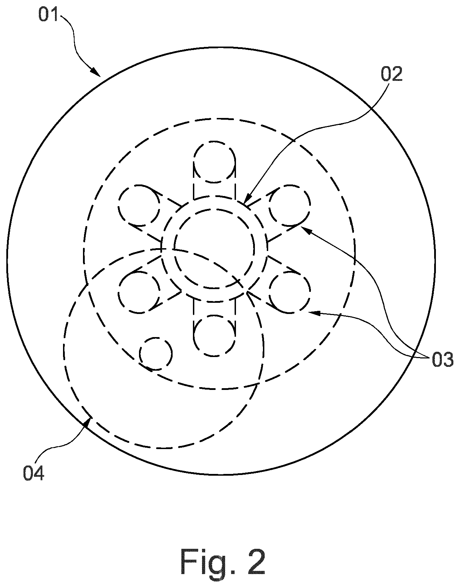

In the embodiment of Fig. 1 and Fig. 2, the first particle separator 03 comprises exit pipes which change the upwards direction of the exit flow from the riser 02 by approximately 180° into a downwards flow direction within the fragmentation reactor 01 and outside the riser 02, which in the present context is referred to as a change of direction particle separator.

In the embodiment of Fig 4, the first particle separator 03 induces a gas particle separation, forcing a tangential, relative to the wall of reactor 01, exit of the riser gas and solids into the reactor 1 and thereby performing the separation. A portion of the particles settles at the bottom part of the fragmentation reactor 01 after exiting the first particle separator 03.

The described features of the riser 02, the position of feedstock inlet 06, and the low residence time first particle separator 03 provide the possibility of a very low contact time between fluidisable particles and feedstock solution depending, of course, also on process parameters such as volume flows and specific dimensions, which are to be adapted to the process demands.

An optional cooling section 05 is arranged within the fragmentation reactor 01. In the embodiment of Fig. 1, the cooling section is between the first particle separator 03 and the outlet O' of the reactor 01.

The fragmentation product is extracted from the fragmentation reactor 01 via the product outlet 09.

In the embodiments shown in Fig. 1 and Fig 4, an optional further second particle separator 04 is provided to separate a further fraction of the fluidisable particles from the product stream before the fragmentation product is extracted.

The second particle separator 04, such as for instance a cyclone, may present a higher separation efficiency than the change of direction separator 03 alone. The gas outlet of the cyclone 04 is connected to the product outlet 09, and the particles from the particle outlet of the cyclone 04 are carried to the bottom of the fragmentation reactor 01, where the fluidisable particles are maintained fluidised by use of fluidisation gas supplied via a secondary fluidisation gas inlet 21. The distribution of fluidisation gas over the horizontal cross section of the vessel 1 is ensured, e.g. using spargers. At the bottom of the fragmentation reactor 01, a particle outlet 10 enables the spent fluidisable particles of the fragmentation reactor 01 to be extracted and carried elsewhere, e.g. for reheating in another reactor. A stripping of product gas (fragmentation product) just before or after the fluidisable particle outlet 10 in figure 1-4 is also envisaged, but not shown on the figures.

Fig. 2 is a top view of the fragmentation reactor of Fig 1. As illustrated, the riser 02 is located in the horizontal cross sectional centre of the fragmentation reactor 01. Furthermore, the plurality of exit pipes forming the first particle separator 03 is shown, as well as the second particle separator 04, which is located off-centre to the fragmentation reactor 01.

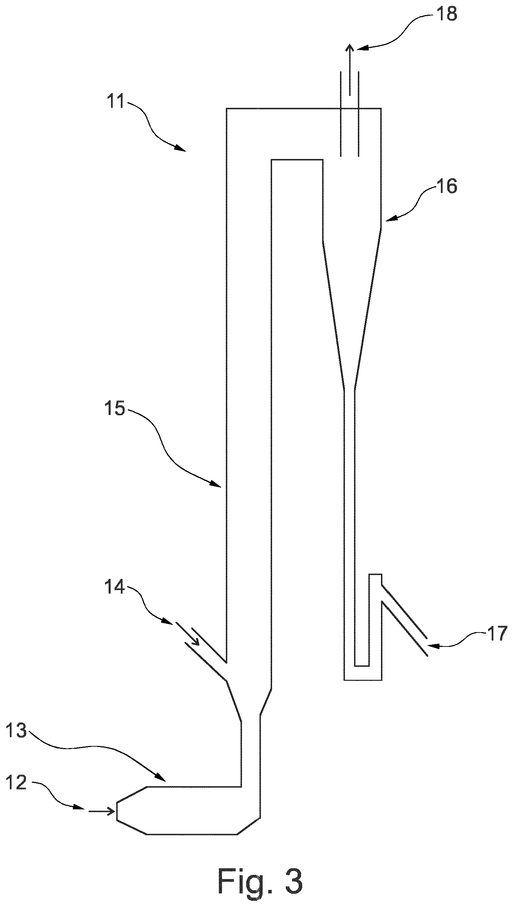

In Fig. 3 and Fig. 4, a reheater 11 for reheating the fluidisable particles exiting the fragmentation reactor 01 is shown. The reheater particle inlet 14 is in fluid connection with the fluidisable particle outlet 10, and the reheater fluidisable particle outlet 17 is in fluid connection with the fluidisable particle inlet 07. The reheater 11 also comprises a riser type fluidised bed, and a reheater riser 15, with a burner chamber 13 arranged in fluid connection to the lower part of the reheater riser 15. A fuel and combustion air inlet 12 enables fuel and combustion air to be provided to the burner chamber 13, which, during operation, provides heat to the reheater riser 15. The reheater fluidisable particle inlet 14 is arranged in the lower part of the reheater riser 15 and enables the fluidisable particles exiting the fragmentation reactor 01 to enter the reheater riser 15 where they are fluidised in an upwards flow by the hot gas provided by the burner chamber 13 while being heated. The connection between the burner chamber 13 and the reheater fluidisable particle inlet 14 is deliberately designed to reduce or prevent fall-through of fluidisable particles from the riser 15 into the burner chamber 13. This design could take many different forms. For example, in Fig 3 and Fig 4, this is illustrated by the constriction between 13 and 15 leading to an increased gas velocity preventing or reducing a fall-through of particles. After reheating, the fluidisable particles are separated from the combustion gas and are fed back to the fragmentation reactor 01. In the embodiment of Fig 3, the reheater particle separator 16 is a cyclone which enables gas to exit the reheater 11 via the reheater gas outlet 18 while the separated fluidisable particles exit the reheater 11 via the reheater fluidisable particle outlet 17 connected to the fluidisable particle outlet of the cyclone of the reheater 11. It is to be understood that the extent of separation in the particle separators depends on various process parameters, such as pressure loss in the separator, flow velocities, particle size etc., as known in the art.

In the embodiment of Fig 4, the reheater first particle separator 16 is similar to the fragmentation reactor first particle separator 03. The embodiment of Fig. 4 is also equipped with a secondary cyclone type particle separator 19. Both particle separators 16, 19 deliver fluidisable particles to the bottom of reheater 11. In the lowermost position of the reheater 11, a section 20 for stripping excess oxygen from the fluidisable particles is placed. Secondary fluidisation and stripping gas inlets 22 for the reheater 11 and the section 20 in the embodiment of Fig 4 are distributed over the cross section using e.g. spargers or other methods. Additional fluidisation gas inlets may be present in the reheater 11 in Fig 4, but are not shown. A stripping of product gas (fragmentation product) just before or after the fluidisable particle outlet 10 in figure 1-4 is also envisaged, but not shown on the figures.

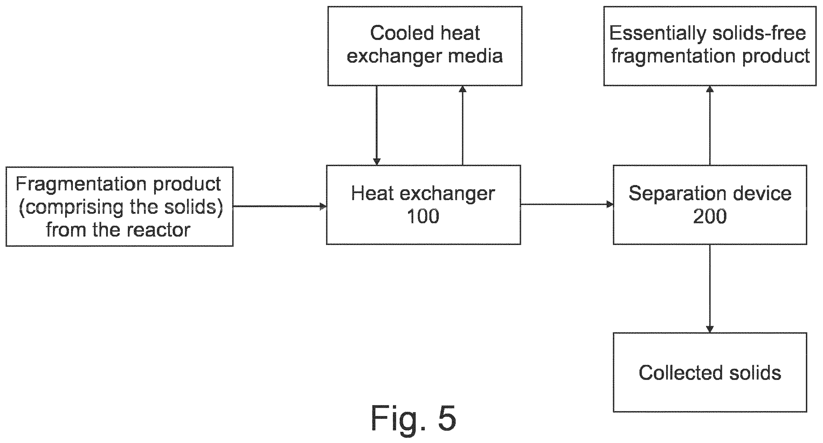

With reference to Fig. 5, further components of the system of Fig. 4 are illustrated (although not all components of the system are illustrated). The system comprises the components of Fig. 4. In other embodiments, the system includes the fragmentation reactor of Fig. 1 instead of that shown in Fig. 4. The system may further comprise a cooling device, such as a heat exchanger 100, for cooling the product gas (fragmentation product). The system also comprises a physical separation device 200 (discussed below). In the embodiment of Fig. 5, the heat exchanger 100 is arranged downstream of the reactor 1, and the physical separation device 200 is arranged downstream of the heat exchanger 100. Those skilled in the art will understand that the system may comprise further components, such as one or more further cooling steps or other unit operations.

In the embodiment of Fig. 5, the product gas traverses the product outlet 09 to the heat exchanger 100, and then to the physical separation device 200, and then to further downstream equipment. In the embodiment of Fig. 5, the physical separation device 200 is a filter, specifically a candle filter wherein the filter element provided by the candles of the candle filter comprised a porous matrix of metal fibres. More specifically, the metal fibres comprised a nickel-chromium-molybdenum alloy (alloy 59).

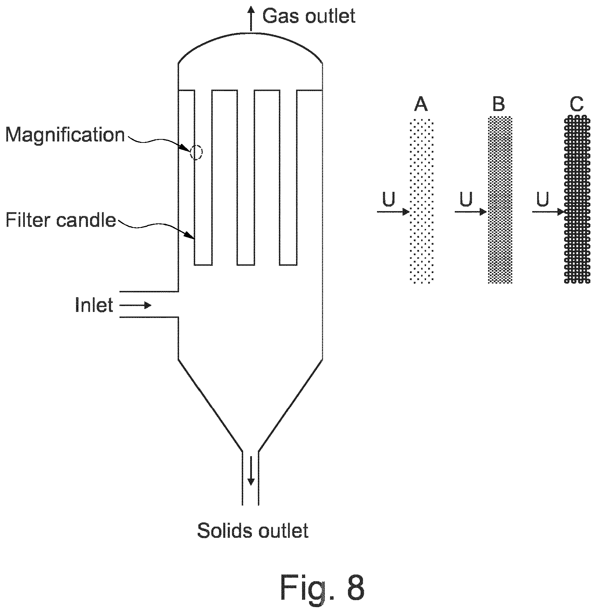

Fig. 8 shows a schematic diagram of the candle filter. The candle filter comprises an enclosure having a solids/fragmentation product inlet through which the fragmentation product comprising the solids are directed, a solids outlet through which separated solids are directed, and a fragmentation product/gas outlet through which the fragmentation product from which solids have been separated is directed. A plurality of candles are arranged within the enclosure. Each candle comprises a filter element for separating the solids from the fragmentation product. Each filter element comprises a filtration surface (or filtration area). The filtration surface may be considered as the interface through which the fragmentation product comprising the solids enters the filter element in use. The filtration surface is the outer surface of the filter element. In use, the fragmentation product enters each filter element via the filtration surfaces, such that the solids are separated from the fragmentation product by each filter element. The separated solids are removed from the enclosure via the solids outlet. The solids outlet may be equipped with a valve system (not shown) to prevent gas from escaping and to remove the collected solids.

In other embodiments, other physical separation devices and other filters are envisaged. For example, candle filters are described in Gasification (Higman, C., 2nd edition, 2008, p. 224-225). The functioning and operation of a candle filter is known to those skilled in the art, and therefore is not described herein in further detail.

In some embodiments (e.g. the embodiment of Fig. 5), the step of cooling the fragmentation product using the heat exchanger 100 is the earliest step of cooling the fragmentation product. In some embodiments, no step of cooling the fragmentation product is performed in the reactor 01. In some embodiments, no step of cooling the fragmentation product is performed prior to the step of cooling the fragmentation product using the heat exchanger 100.

In this particular embodiment, prior to traversing the product outlet 09, the fragmentation product passes through the first particle separator 03 and the second particle separator 04 (as discussed above). These particle separators 03, 04 reduce the amount of the heat carrying particles in the fragmentation product.

The separation device 200 separates solids from the fragmentation product, wherein the solids comprise solids selected from the fluidisable heat carrying particles, fragments of the fluidisable heat carrying particles, by-products from the thermolytic fragmentation of the feedstock solution (the latter is discussed below), and mixtures thereof. In this particular embodiment, the solids consisted essentially of solids selected from the fluidisable heat carrying particles, fragments of the fluidisable heat carrying particles, by-products from the thermolytic fragmentation of the feedstock solution, and mixtures thereof. By separating the solids from the fragmentation product, these solids have a reduced impact on equipment downstream of the reactor 1. In use, the fragmentation product exits the reactor 01 via the product outlet 09 and passes through the heat exchanger 100 before passing through the separation device 200.

The heat exchanger 100 was provided at ground level, whereas the reactor 01 was elevated relative to the ground level.

As will be understood by those skilled in the art, the water may be introduced into the reactor by various means, including, for example, via the feedstock solution, the fluidisation gas, and/or the atomisation gas. However, it is to be understood that the fluidisation gas and/or the atomisation gas may comprise inert gases, such as $N_2$, CO, $CO_2$, $CH_4$, or mixtures thereof.

Example 1

Thermolytic fragmentation of aqueous feedstock solution (glucose syrup) comprising approximately 60 wt.% glucose and 40 wt.% water (based on the total weight of the feedstock solution) was conducted in a system according to an embodiment of the present disclosure (i.e. Fig. 4 but wherein the optional cooling step 05 was omitted). The aqueous feedstock solution was fed into the reactor at a rate of 15 kg/h.

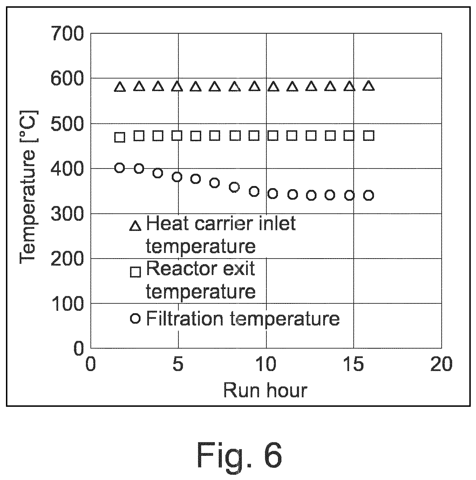

The fragmentation reactor 1 was operated at a pressure around 2.5 bara (absolute) (at the outlet O' of the reactor) with an inlet 07 heat carrier temperature of approximately 580°C and a reactor outlet O' temperature of approximately 475°C. Over the duration of the reactor 01 run time of around 15 hours, the temperature of the filter 200 (e.g. at an inlet of the filter 200) was gradually decreased from approximately 400°C to 340°C, by increasing the cooling rate of the indirect cooler heat exchanger 100 positioned between the reactor 01 and the filter 200. Figure 6 shows the temperature profiles during this 15-hour run. The feedstock solution was injected into the reactor 1 using a gas assisted internal mix atomisation nozzle (i.e. the feedstock and atomisation gas inlet 08) entering from the base of the riser 02, with an atomisation gas flow of 15 kg/h of nitrogen, fluidisation steam gas flow of 7 kg/h of steam through 6, and 12 kg/h of steam added through inlet 21.

The average gas residence time (mean residence time of the fragmentation product) in the filter 200 was approximately 16-17 seconds (although those skilled in the art understand that this varies with temperature). The surface filtration superficial velocity (i.e. the superficial velocity at the filtration surface of the filter) was from 0.42 to 0.46 cm/s.

With reference to Fig. 8, as the fragmentation product comprising the solids flows to the candles in use, the fragmentation product comprising the solids is distributed over the candles, and flows through the filter element of the candles. The fragmentation product enters the filter element of the candles via the filtration surface of the candles. The fragmentation product comprising the solids flows through the filtration surface of the candles at an average superficial velocity, which may be calculated by:

$$U = Q/Area$$

where Q is the actual volumetric gas in $m^3/s$ flow and Area is the area of the filtration surface in $m^2$. Those skilled in the art would know how to calculate the actual gas flow and correct it according to pressure and temperature. The filter element of the candles can be made of different materials and have different porosities, as illustrated in A, B or C in the right hand side of Fig. 8. The gas velocity, U, at the filtration surface will be approximately independent of material type.

Following filtration at the filter 200, a solids-lean fragmentation product was obtained. The solids-lean fragmentation product was essentially free of the solids.

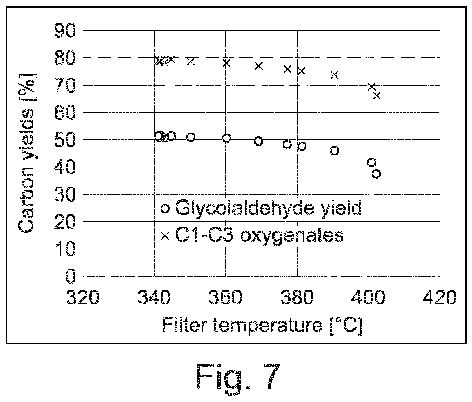

Following filtration at the filter 200, a stream of the fragmentation product (solids-lean) was condensed. The glycolaldehyde concentration of the fragmentation product (solids-lean) was measured by HPLC at different times during the 15-hour run. Also, the concentration of C1-C3 oxygenates of the fragmentation product (solids-lean) was measured by HPLC at different times during the 15-hour run. The yield of C1-C3 oxygenates (i.e. all C1-C3 oxygenates) was determined. Using water as inert reference, a carbon based yield or recovery of glycolaldehyde was calculated for each filtration temperature. The recovery or yield of glycolaldehyde is the percentage of carbon in the feedstock solution that is recovered as carbon in glycolaldehyde. The same calculations were performed for all C1-C3 oxygenates. Fig. 7 shows the carbon yield or recovery of glycolaldehyde and of C1-C3 oxygenates (all C1-C3 oxygenates) as a function of filtration temperature. It was observed that the glycolaldehyde yield increased significantly with decreasing filter temperature, i.e. from below 40% to around 50%. It also was observed that the C1-C3 oxygenates yield increased significantly with decreasing filter temperature, i.e. from around 65% to around 78%.

The present inventors therefore have surprisingly discovered that the yield of C1-C3 oxygenates (e.g. glycolaldehyde) is dependent on filtration temperature, which is preferably below 370°C and more preferably below 350°C.

The present inventors therefore have surprisingly discovered that by cooling the fragmentation product downstream of the reactor 01 to the cooling temperature, the process provides for improvements in efficiency and design, whilst achieving a good product yield of C1-C3 oxygenates (e.g. glycolaldehyde). In particular, by virtue of the downstream cooling step, e.g. rather than cooling within the reactor, the cooling heat exchanger 100 and the reactor 01 can be arranged in different locations from each other. In this way, the reactor 01 can be designed without the need to accommodate the cooling heat exchanger 100, which reduces design complexity and installation complexity, and improves space-efficiency and ease of handling. With regard to reducing installation complexity, the cooling heat exchanger 100 can be located closer to ground level than the reactor 01 and installed independently of the reactor 01. With regard to space-efficiency and ease of handling, as the reactor 01 can be designed without the need to accommodate the cooling heat exchanger 100, the reactor 01 can be more compact and of a lower mass. Moreover, for example, the cooling heat exchanger 100 can be connected to the reactor 01 (e.g. in a modular fashion) once the reactor 01 has been installed. The cooling to the cooling temperature downstream of the reactor 01 unexpectedly led to these advantages as well as a good product yield of C1-C3 oxygenates.

The afore-mentioned advantages are particularly beneficial in the context of industrial production, where significant capital expenditure (CAPEX) and operational expenditure (OPEX) can be used for building and maintaining process equipment and in which product yield is a primary consideration.

Example 2

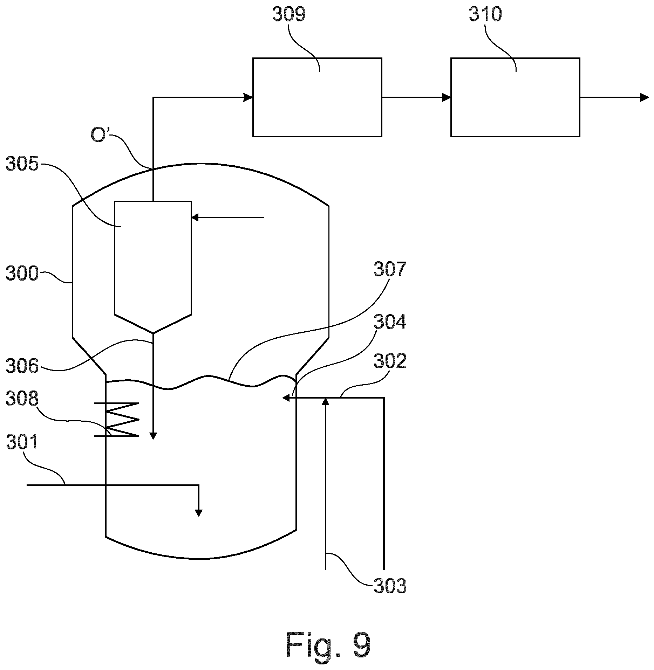

This example illustrates another aspect of performing thermolytic fragmentation of sugars in an industrial setting according to the invention. Referring to Fig. 9, the system and process uses a fluidised bed fragmentation reactor (300). Inside the reactor (300), a fluidisation gas (301) is added by a gas distributor (not shown in detail) in the bottom of the reactor (300), for fluidisation of heat carrying particles used for thermolytic fragmentation of the sugars. The gas distributor distributes the fluidisation gas (301) over the entire cross section of the reactor (300). In Fig. 9, the gas distributor is a gas sparger having a downward direction (although other directions such as upward, horizontal or therebetween are also a possibility). Liquid sugar feedstock (302) is mixed with atomisation gas (303) in feed nozzle (304) to provide a fine spray of liquid droplets before contacting the heat carrying particles in the reactor (300). Only one feed nozzle (304) is shown in Fig. 9 but it is to be understood that multiple feed nozzles can be used and placed at different positions within the reactor (300) to provide a good distribution of feed (302) over the reactor (300).

Liquid sugar feedstock (302) thus brought into contact with the heat carrying particles undergo cracking within the reactor and a gas phase C1-C3 oxygenate rich fragmentation product is obtained. The cracking and feed water evaporation increases the gas volume flow thus increasing the upward gas flow rate. The gas flows upwards and into a cyclone (305) (the first particle separator). Inside the cyclone (305), most (e.g. at least 95 wt.%) of the entrained heat carrying particles are separated and recycled (306) to the lower part of the reactor (300). The outlet of the reactor O' conveys the fragmentation product gas to downstream processing. It is to be understood that the particle separator (305) can be configured in different ways and multiple particle separators may be used. For example, parallel cyclones, or cyclones in series, or a combination thereof may be used. A surface filtration filter may also be used.

The lower part of reactor (300) operates in a dense type of fluidised bed with superficial gas velocities below approximately 2 m/s (meters per second). The upper part of reactor 300 operates in a lean type of phase with superficial gas velocities above approximately 3 m/s. The demarcation between the mode of operation (dense or lean) is not sharp cut and may also be dependent on gas and solid physical properties such as densities, gas viscosity and particle size and shape. The increase in gas flow rate from the cracking process may also depend on feed concentration and product yields. In Fig. 9, the approximate boundary separating the dense phase and the lean phase is indicated by the curved line (307). The curved line (307) also shows the approximate position of the top surface of the dense fluidised bed of heat carrying particles.

Inside the dense fluidised bed of heat carrying particles, an indirect heating arrangement (308) is positioned to bring the energy required for the thermolytic fragmentation step and to account for heat losses and product gas losses. The heating arrangement (308) could be resistive electrical heating rods or other types or indirect heating methods. The dense mode of fluidisation ensures good mixing and transport of the heat carrying particles in the reactor (300).

The heating arrangement (308) could also be positioned externally to reactor (300), and additional means for transferring of the heat carrying particles between the reactor (300) and the heating arrangement (308) may be provided. This configuration is not shown.

As shown in Fig. 9, downstream the outlet O' of the reactor (300), there is a cooling step (309) followed by a solid separation step (310).

The system of Fig. 9 may be used for performing thermolytic fragmentation of sugars under industrial conditions. To the reactor (300) is added approximately 25000 liters (bulk) of heat carrying particles as described in Example 2 of WO 2021/032590 A1. An aqueous solution of 60 wt.% glucose (302) is fed into the reactor (300) at a rate of 23000 kg/h (kilograms per hour). Steam as the atomisation gas (303) is fed into the reactor (300) at a rate of 1380 kg/h. Steam as the fluidisation gas (301) is fed into the reactor (300) at a rate of 5741 kg/h. The cooling step (309) involves cooling the fragmentation product to a cooling temperature of from 230°C to 390°C. The separation step (310) involves separating solids from the fragmentation product cooled to the cooling temperature, wherein the solids comprise solids selected from the fluidisable heat carrying particles, fragments of the fluidisable heat carrying particles, by-products from the thermolytic fragmentation of the feedstock solution, and mixtures thereof. The following yields of main C2-C3 oxygenates on carbon basis from glucose are obtained: 57 C-% glycolaldehyde, 10 C-% pyruvaldehyde, 4 C-% acetol, 3 C-% glyoxal, and 10 C-% formaldehyde.

The product gas flow at the outlet O' of the reactor (300) is approximately 24380 Nm3/h (Nm3 refers to normal cubic meters at a temperature of 0°C and a pressure of 101.325 kPa (1 atmosphere absolute)).

Yields of other side products, such as CO, CO2, acetic acid may not change this flow rate significantly. It is to be understood that, for example, varying the flow rate and/or composition of the atomisation gas (303) or fluidisation gas (301) may change the resulting gas flow at the outlet O'. The temperature of the process gas at the outlet O' of the reactor (300) is approximately 525°C and cooling this gas to a cooling temperature of 300°C results in a required cooling duty of approximately 3.7 MW (Mega watts). The reactor (300) is operated at a pressure of approximately 212.782 kPa (2.1 atmosphere absolute) immediately before the gas inlet to the particle separator (305).

Those skilled in the art will appreciate that the present invention may be scaled-up as desired. The present invention is suitable for industrial scale production.

## Claims

1. A process for thermolytic fragmentation of a sugar into C1-C3 oxygenates, the process comprising the steps of:

   a) providing an aqueous feedstock solution comprising the sugar;
   b) providing a fluidised bed fragmentation reactor for thermolytically fragmenting the sugar and comprising fluidisable heat carrying particles;
   c) introducing the feedstock solution into the reactor to thermolytically fragment the sugar to provide a fragmentation product comprising the C1-C3 oxygenates, wherein the temperature of the fragmentation product at an outlet of the reactor is at least 400°C;
   d) cooling the fragmentation product downstream of the reactor to a cooling temperature of from 230°C to 390°C; and
   e) separating solids from the fragmentation product cooled to the cooling temperature, wherein the solids comprise solids selected from the fluidisable heat carrying particles, fragments of the fluidisable heat carrying particles, by-products from the thermolytic fragmentation of the feedstock solution, and mixtures thereof.

2. The process according to claim 1, wherein step d) comprises indirectly cooling the fragmentation product to the cooling temperature, optionally wherein step d) comprises cooling the fragmentation product to the cooling temperature using a cooling device, optionally wherein the cooling device is a heat exchanger.

3. The process according to claim 1 or 2, wherein step d) is the earliest step of cooling the fragmentation product, optionally wherein no step of cooling the fragmentation product is performed within the reactor, optionally wherein no step of cooling the fragmentation product is performed prior to step d).

4. The process according to any one of claims 1 to 3, wherein the cooling temperature is from 260 to 385°C, such as from 270 to 380°C, from 300 to 375°C, or from 330 to 370°C.

5. The process according to any one of claims 1 to 4, wherein the mean residence time of the fragmentation product between step d) and step e) is no greater than 50 s.

6. The process according to any one of claims 1 to 5, wherein the mean residence time of the fragmentation product in step e) is from 8 s to 34 s.

7. The process according to any one of claims 1 to 6, wherein step e) comprises separating the solids from the fragmentation product using a physical separation device.

8. The process according to claim 7, wherein the physical separation device is a filter, optionally wherein the filter has an efficiency of at least 99.9% for particles having a size of at least 50 micrometres, such as at least 30 micrometres, at least 20 micrometres, or at least 10 micrometres, optionally wherein the filter is selected from a bag filter and a candle filter.

9. The process according to claim 8, wherein the filter comprises a filter element having a filtration surface, wherein the superficial velocity of the fragmentation product at the filtration surface is from 0.1 to 3 cm/s.

10. The process according to any one of claims 1 to 9, wherein the sugar is a carbohydrate comprising one or more C6 saccharide units and/or C5 saccharide units, and/or wherein the sugar is a monosaccharide or a disaccharide, optionally wherein the sugar is selected from sucrose, lactose, xylose, arabinose, ribose, mannose, tagatose, galactose, glucose, and fructose, and/or wherein the total sugar content in the feedstock solution is from 30 to 99 wt.%, such as from 40 to 90 wt.%, or from 50 to 80 wt.%, based on the total weight of the feedstock solution.

11. The process according to any one of claims 1 to 10, wherein the fragmentation product comprises glycolaldehyde; optionally wherein the fragmentation product comprises one, more, or each of formaldehyde, glycolaldehyde, glyoxal, pyruvaldehyde, and acetol; optionally wherein the fragmentation product comprises glycolaldehyde in an amount of at least 10 wt.% based on the total weight of the fragmentation product.

12. The process according to any one of claims 1 to 11, wherein step d) is performed at a location below the location at which the reactor is provided.

13. The process according to any one of claims 1 to 12, wherein the pressure at the outlet of the reactor is at least 1.5 bara.

14. The process according to any one of claims 1 to 13, wherein the fragmentation reactor comprises a riser.

15. The process according to any one of claims 1 to 14, comprising one or more further separation steps between step c) and step d), wherein the or each separation between step c) and step d) comprises separating solids from the fragmentation product, wherein the solids comprise solids selected from fluidisable heat carrying particles, fragments of the fluidisable heat carrying particles, by-products from the thermolytic fragmentation of the feedstock solution, and mixtures thereof, wherein the or each separation step between step c) and step d) comprises separating at least 50 wt.% of the solids from the fragmentation product based on the total weight of the solids.

**Patentansprüche**

1. Prozess zur thermolytischen Fragmentierung eines Zuckers in C1-C3-Oxygenate umfasst, wobei der Prozess die folgenden Schritte umfasst:

   a) Bereitstellen einer wässrigen Ausgangslösung, die den Zucker umfasst;
   b) Bereitstellen eines Wirbelschichtfragmentierungsreaktors zur thermolytischen Fragmentierung des Zuckers und der fluidisierbare, wärmetragende Partikel umfasst;
   c) Einführen der Ausgangslösung in den Reaktor zur thermolytischen Fragmentierung des Zuckers, um ein Fragmentierungsprodukt bereitzustellen, das die C1-C3-Oxygenate umfasst, wobei die Temperatur des Fragmentierungsprodukts am Auslass des Reaktors mindestens 400°C beträgt;
   d) Kühlen des Fragmentierungsprodukts stromabwärts des Reaktors auf eine Abkühltemperatur von 230°C bis 390°C; und
   e) Abtrennen von Feststoffen aus dem auf die Abkühltemperatur abgekühlten Fragmentierungsprodukt, wobei die Feststoffe Feststoffe umfassen, die ausgewählt sind aus den fluidisierbaren wärmetragenden Partikeln, Fragmenten der fluidisierbaren wärmetragenden Partikel, Nebenprodukten aus der thermolytischen Fragmentierung der Ausgangslösung und Mischungen davon.

2. Prozess nach Anspruch 1, wobei Schritt d) das indirektes Abkühlen des Fragmentierungsprodukts auf die Abkühltemperatur umfasst, optional wobei Schritt d) Abkühlen des Fragmentierungsprodukts auf die Kühltemperatur unter Verwendung einer Kühlvorrichtung umfasst, optional wobei es sich bei der Kühlvorrichtung um einen Wärmetauscher handelt.

3. Prozess nach Anspruch 1 oder 2, wobei Schritt d) der früheste Schritt des Abkühlens des Fragmentierungsprodukts ist, optional wobei kein Kühlschritt des Fragmentierungsprodukts innerhalb des Reaktors durchgeführt wird, optional wobei vor Schritt d) kein Schritt des Abkühlens des Fragmentierungsprodukts durchgeführt wird.

4. Prozess nach einem der Ansprüche 1 bis 3, wobei die Abkühltemperatur im Bereich von 260 bis 385°C, beispielsweise im Bereich von 270 bis 380°C, von 300 bis 375°C oder von 330 bis 370°C liegt.

5. Prozess nach einem der Ansprüche 1 bis 4, wobei die mittlere Verweilzeit des Fragmentierungsprodukts zwischen Schritt d) und Schritt e) nicht größer als 50 s ist.

6. Prozess nach einem der Ansprüche 1 bis 5, wobei die mittlere Verweilzeit des Fragmentierungsprodukts in Schritt e) zwischen 8 s und 34 s liegt.

7. Prozess nach einem der Ansprüche 1 bis 6, wobei Schritt e) Abtrennen der Feststoffe aus dem Fragmentierungsprodukt unter Verwendung einer physikalischen Trennvorrichtung umfasst.

8. Prozess nach Anspruch 7, wobei es sich bei der physikalischen Trennvorrichtung um einen Filter handelt, optional wobei der Filter einen Wirkungsgrad von mindestens 99,9 % für Partikel aufweist, die eine Größe von mindestens 50 Mikrometern, wie beispielsweise mindestens 30 Mikrometern, mindestens 20 Mikrometern oder mindestens 10 Mikrometern aufweisen, optional wobei der Filter aus einem Beutelfilter und einem Kerzenfilter ausgewählt ist.

9. Prozess nach Anspruch 8, wobei der Filter ein Filterelement umfasst, der eine Filtrationsfläche aufweist, wobei die Oberflächengeschwindigkeit des Fragmentierungsprodukts an der Filtrationsfläche zwischen 0,1 und 3 cm/s liegt.

10. Prozess nach einem der Ansprüche 1 bis 9, wobei es sich bei dem Zucker um ein Kohlenhydrat handelt, das eine oder mehrere C6-Saccharideinheiten und/oder C5-Saccharideinheiten umfasst, und/oder wobei es sich bei dem Zucker um ein Monosaccharid oder ein Disaccharid handelt, optional wobei der Zucker ausgewählt ist aus Saccharose, Lactose, Xylose, Arabinose, Ribose, Mannose, Tagatose, Galactose, Glucose und Fructose, und/oder wobei der Gesamtzuckergehalt in der Ausgangslösung im Bereich von 30 bis 99 Gew.-% liegt, zum Beispiel von 40 bis 90 Gew.-% oder von 50 bis 80 Gew.-%, basierend auf dem Gesamtgewicht der Ausgangslösung.

11. Prozess nach einem der Ansprüche 1 bis 10, wobei das Fragmentierungsprodukt Glycolaldehyd umfasst; optional wobei das Fragmentierungsprodukt eines, mehrere oder jedes umfasst von Formaldehyd, Glycolaldehyd, Glyoxal, Pyruvaldehyd und/oder Acetol; optional wobei das Fragmentierungsprodukt Glycolaldehyd in einer Menge von mindestens 10 Gew.-% bezogen auf das Gesamtgewicht des Fragmentierungsprodukts, umfasst.

12. Prozess nach einem der Ansprüche 1 bis 11, wobei Schritt d) an einer Stelle unterhalb der Stelle, an der der Reaktor bereitgestellt ist, durchgeführt wird.

13. Prozess nach einem der Ansprüche 1 bis 12, wobei der Druck am Auslass des Reaktors mindestens 1,5 bar beträgt.

14. Prozess nach einem der Ansprüche 1 bis 13, wobei der Fragmentierungsreaktor ein Steigrohr umfasst.

15. Prozess nach einem der Ansprüche 1 bis 14, umfassend einen oder mehrere weitere Trennschritte zwischen Schritt c) und Schritt d), wobei der oder jeder Trennschritt zwischen Schritt c) und Schritt d) Abtrennen von Feststoffen aus dem Fragmentierungsprodukt umfasst, wobei die Feststoffe Feststoffe umfassen, die ausgewählt sind aus fluidisierbaren wärmetragenden Partikeln, Fragmenten der fluidisierbaren wärmetragenden Partikel, Nebenprodukten aus der thermolytischen Fragmentierung der Ausgangslösung und Mischungen davon, wobei der oder jeder Trennschritt zwischen Schritt c) und Schritt d) Abtrennen von mindestens 50 Gew.-% der Feststoffe aus dem Fragmentierungsprodukt basierend auf dem Gesamtgewicht der Feststoffe, umfasst.

**Revendications**

1. Processus de fragmentation thermolytique d'un sucre en composés oxygénés en C1-C3, le processus comprenant les étapes consistant à :

  a) fournir une solution aqueuse d'alimentation comprenant le sucre ;
  b) fournir un réacteur de fragmentation à lit fluidisé pour la fragmentation thermolytique du sucre et comprenant des particules thermosensibles fluidisables ;
  c) introduire la solution d'alimentation dans le réacteur pour fragmenter thermolytiquement le sucre afin de fournir un produit de fragmentation comprenant les composés oxygénés en C1-C3, dans lequel la température du produit de fragmentation à la sortie du réacteur est d'au moins 400 °C ;
  d) refroidir le produit de fragmentation en aval du réacteur à une température de refroidissement comprise entre 230 °C et 390 °C ; et
  e) séparer les solides du produit de fragmentation refroidi à la température de refroidissement, dans lequel les solides comprennent des solides sélectionnés parmi les particules thermosensibles fluidisables, des fragments de particules thermosensibles fluidisables, des sous-produits de la fragmentation thermolytique de la solution d'alimentation et des mélanges de ceux-ci.

2. Processus selon la revendication 1, dans lequel l'étape d) comprend le refroidissement indirect du produit de fragmentation à la température de refroidissement, optionnellement dans lequel l'étape d) comprend le refroidissement du produit de fragmentation à la température de refroidissement à l'aide d'un dispositif de refroidissement, optionnellement dans lequel le dispositif de refroidissement est un échangeur de chaleur.

3. Processus selon la revendication 1 ou 2, dans lequel l'étape d) est la première étape de refroidissement du produit de fragmentation, éventuellement dans lequel aucune étape de refroidissement du produit de fragmentation n'est effectuée à l'intérieur du réacteur, éventuellement dans lequel aucune étape de refroidissement du produit de fragmentation n'est effectuée avant l'étape d).

4. Processus selon l'une quelconque des revendications 1 à 3, dans lequel la température de refroidissement est de 260 à 385 °C, par exemple de 270 à 380 °C, de 300 à 375 °C ou de 330 à 370 °C.

5. Processus selon l'une quelconque des revendications 1 à 4, dans lequel le temps de séjour moyen du produit de fragmentation entre l'étape d) et l'étape e) n'est pas supérieur à 50 s.

6. Processus selon l'une quelconque des revendications 1 à 5, dans lequel le temps de séjour moyen du produit de fragmentation à l'étape e) est de 8 s à 34 s.

7. Processus selon l'une quelconque des revendications 1 à 6, dans lequel l'étape e) comprend la séparation des solides du produit de fragmentation à l'aide d'un dispositif de séparation physique.

8. Processus selon la revendication 7, dans lequel le dispositif de séparation physique est un filtre, optionnellement dans lequel le filtre présente une efficacité d'au moins 99,9 % pour les particules présentant une taille d'au moins 50 micromètres, telles que d'au moins 30 micromètres, d'au moins 20 micromètres ou d'au moins 10 micromètres, optionnellement dans lequel le filtre est choisi parmi un filtre à sac et un filtre à bougie.

9. Processus selon la revendication 8, dans lequel le filtre comprend un élément filtrant présentant une surface de filtration, dans lequel la vitesse superficielle du produit de fragmentation à la surface de filtration est comprise entre 0,1 et 3 cm/s.

10. Processus selon l'une quelconque des revendications 1 à 9, dans lequel le sucre est un glucide comprenant une ou plusieurs unités saccharidiques en C6 et/ou unités saccharidiques en C5, et/ou dans lequel le sucre est un monosaccharide ou un disaccharide, éventuellement dans lequel le sucre est choisi parmi le saccharose, le lactose, le xylose, l'arabinose, le ribose, le mannose, le tagatose, le galactose, le glucose et le fructose, et/ou dans lequel la teneur totale en sucre de la solution d'alimentation est de 30 à 99 % en poids, par exemple de 40 à 90 % en poids, ou de 50 à 80 % en poids, sur la base du poids total de la solution d'alimentation.

11. Processus selon l'une quelconque des revendications 1 à 10, dans lequel le produit de fragmentation comprend du glycolaldéhyde ; optionnellement dans lequel le produit de fragmentation comprend un, plusieurs ou chacun des composés suivants : formaldéhyde, glycolaldéhyde, glyoxal, pyruvaldéhyde et acétol ; optionnellement dans lequel le produit de fragmentation comprend du glycolaldéhyde en une quantité d'au moins 10 % en poids sur la base du poids total du produit de fragmentation.

12. Processus selon l'une quelconque des revendications 1 à 11, dans lequel l'étape d) est effectuée à un emplacement situé en dessous de l'emplacement où le réacteur est prévu.

13. Processus selon l'une quelconque des revendications 1 à 12, dans lequel la pression à la sortie du réacteur est d'au moins 1,5 bara.

14. Processus selon l'une quelconque des revendications 1 à 13, dans lequel le réacteur de fragmentation comprend une colonne montante.

15. Processus selon l'une quelconque des revendications 1 à 14, comprenant une ou plusieurs étapes de séparation supplémentaires entre l'étape c) et l'étape d), dans lequel la ou chaque séparation entre l'étape c) et l'étape d) comprend la séparation des solides du produit de fragmentation, dans lequel les solides comprennent des solides sélectionnés parmi des particules thermosensibles fluidisables, des fragments de particules thermosensibles fluidisables, des sous-produits de la fragmentation thermolytique de la solution d'alimentation et des mélanges de ceux-ci, dans lequel la ou chaque étape de séparation entre l'étape c) et l'étape d) comprend la séparation d'au moins 50 % en poids des solides du produit de fragmentation sur la base du poids total des solides.

Fig. 1

Fig. 2

Fig. 3

Fig. 4

Fig. 5

Fig. 6

Fig. 7

Fig. 8

Fig. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5397582 A, Underwood
- US 7094932 B, Majerski
- WO 2014131764 A
- WO 2012115754 A
- US 5302280 A, Lomas
- WO 2017216311 A, Larsen
- WO 2021032590 A1

**Non-patent literature cited in the description**

- **HIGMAN, C.** Gasification. 2008, 224-225